# FASCICULE DE BREVET EUROPEEN

(11) **EP 2 015 711 B1**
(45) Date de publication et mention de la délivrance du brevet: **04.12.2013**
(21) Numéro de dépôt: 06755133.3
(22) Date de dépôt: 10.05.2006
(51) Int. Cl.: A61F 2/60, A61F 2/64, A61F 2/68

(54) **APPAREIL DE LIAISON ARTICULEE POUR PROTHESE DE MEMBRE INFERIEUR**
GELENKIGE VERBINDUNGSVORRICHTUNG FÜR BEINPROTHESEN
HINGED CONNECTING APPARATUS FOR A LOWER LIMB PROSTHESIS

(43) Date de publication de la demande: 21.01.2009
(73) Titulaire: S & S Sàrl, 2740 Moutier (CH)
(72) Inventeur: STEINER, Jean-Louis, CH-2740 Moutier (CH); SCHNEIDER, Clément, CH-2525 Le Landeron (CH)
(74) Mandataire: Scheuzger, Beat Otto
(86) Numéro de dépôt international: PCT/EP2006/062217
(87) Numéro de publication internationale: WO 2007/128351

(56) Documents cités:
- EP-A2- 0 549 855
- WO-A-01/37763
- DE-A1- 3 028 608
- DE-C- 325 579
- DE-C- 880 191
- FR-A1- 2 741 526
- GB-A- 1 007 431
- US-A- 2 170 580
- US-A- 4 179 759

## Description

L'invention se rapporte à un appareil de liaison articulée pour une prothèse de membre inférieur.

L'invention se rapporte également à une prothèse équipée de l'appareil de liaison précité.

Une prothèse de membre inférieur avec liaison articulée comprend généralement :
- une première partie capable d'assurer une fonction de cuisse,
- une seconde partie capable d'assurer une fonction de jambe,
- une troisième partie capable d'assurer une fonction de genou,
- une quatrième partie capable d'assurer une fonction de pied en vue de l'appui sur un support.

L'invention peut être utilisée que le genou soit de type monocentrique, c'est-à-dire avec un seul axe d'articulation, ou polycentrique, c'est-à-dire avec plusieurs axes d'articulation.

L'appareil de liaison articulé est incorporé à la troisième partie de manière à assurer la liaison articulée entre la première partie et la seconde partie, et ce, entre au moins deux positions relatives dont :
- une première position dans laquelle la première partie et la seconde partie s'étendent sensiblement dans le prolongement l'une de l'autre, formant un premier angle, et
- une seconde position dans laquelle la première partie et la seconde partie forment un second angle prédéterminé correspondant à la flexion maximale admise par la prothèse.

L'appareil de liaison articulé comprend un premier dispositif destiné à opposer une résistance prédéterminée au moins lors de la flexion de la prothèse, cette résistance étant commutée par ledit premier dispositif entre deux valeurs dont :
- une valeur maximale prédéterminée sélectionnée par défaut, et
- une valeur minimale prédéterminée sélectionnée après que, la première partie et la seconde partie étant situées dans une troisième position voisine de la première position, ces parties sont sollicitées vers la première position et déplacées en cette première position.

Lorsque la prothèse est portée par une personne, c'est-à-dire que la première partie est reliée à une partie subsistante de cuisse, la personne doit pouvoir modifier la configuration de la prothèse et notamment :
- lever la première partie pour contraindre la prothèse à fléchir et tendre à placer ladite première partie et la seconde partie dans la seconde position,
- appuyer sur la première partie pendant que la quatrième partie est en contact avec un support et tendre à placer ladite première partie et la seconde partie dans la première position.

GB1007431 divulgue une jambe artificielle qui a des parties de jambe supérieure et inférieure interconnectées de manière pivotante à l'articulation du genou et des moyens associés à l'articulation du genou pour redresser la jambe après qu'une position courbée prédéterminée a été atteinte, les moyens étant rendus inopérants par le mouvement de pivotement du pied par rapport à la partie de jambe inférieure.

US2170580 se rapporte en général à des membres artificiels, et en particulier à ce type de membre spécialement adapté à des cas où la jambe du porteur a été amputée en-dessus du genou. Elle permet au porteur de rapprocher les mouvements naturels de marche et est de telle nature que les parties de membre seront assemblées pendant le pas en avant du porteur et avant que tout poids soit placé sur cela, où l'effondrement à l'articulation du genou est impossible dans l'action normale de marche.

Les appareils de liaison articulée connus ont leurs avantages, mais on leur reproche de ne pas assurer la complète sécurité de la personne qui les porte, notamment, lorsque cette personne doit descendre une surface inclinée, une marche ou un escalier.

Un résultat que l'invention vise à obtenir est précisément un appareil qui permet de remédier à cet inconvénient.

A cet effet, l'invention a pour objet un appareil de liaison articulée selon la revendication 1.

L'invention a également pour objet une prothèse de membre inférieur équipée de l'appareil de liaison précité.

L'invention sera bien comprise à la lecture de la description ci-après faite à titre d'exemple non limitatif en regard du dessin ci-annexé qui représente schématiquement :

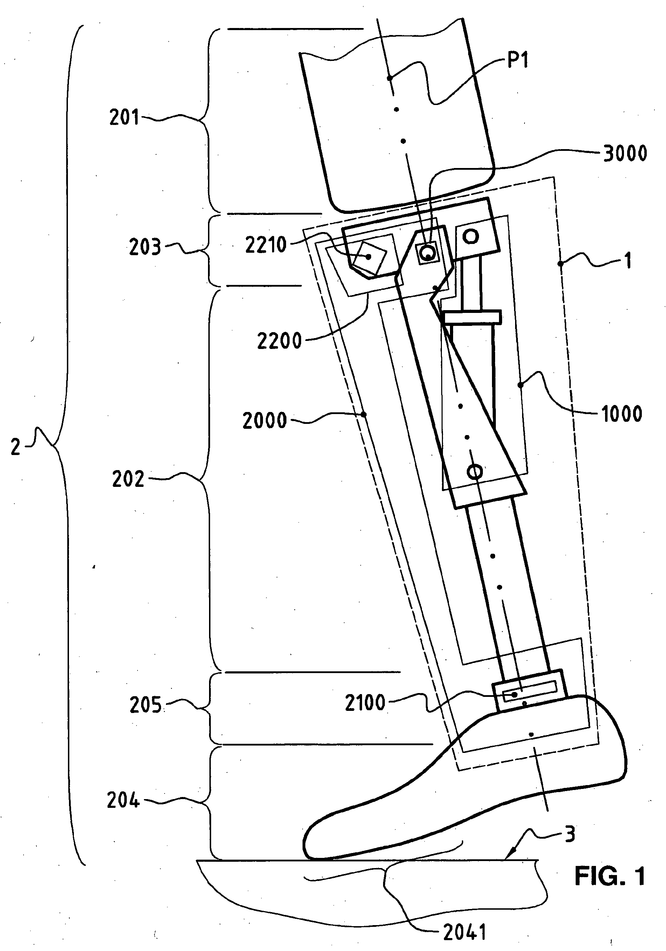

Figures 1 à 3 : des vues de profils d'une prothèse de membre inférieur dans différentes configurations de fonctionnement,

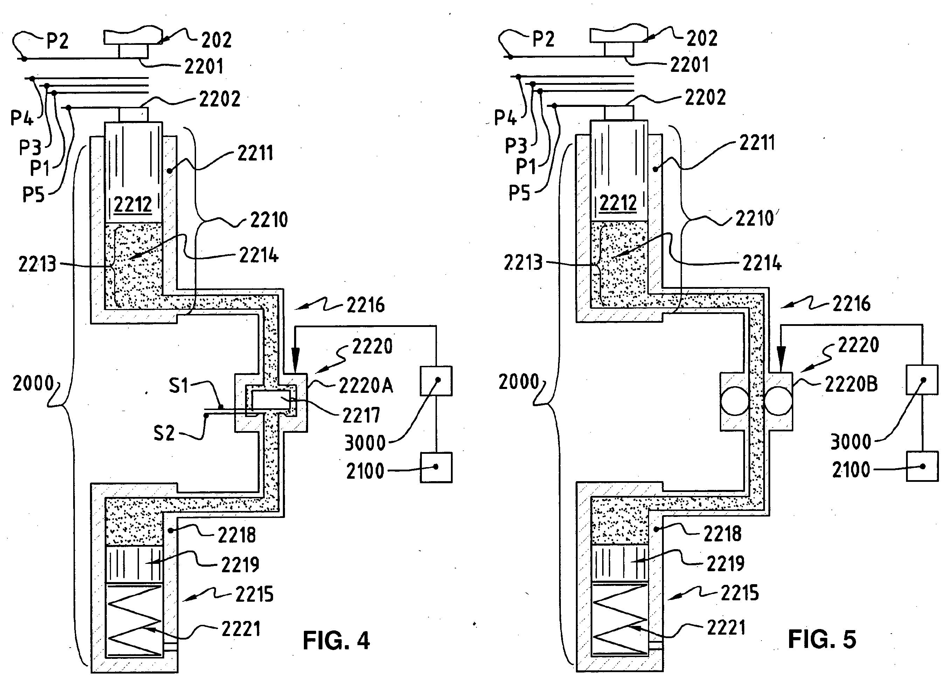

Figures 4 et 5 : vue en coupe, un dispositif utilisé dans l'appareil selon l'invention, selon deux formes de réalisation,

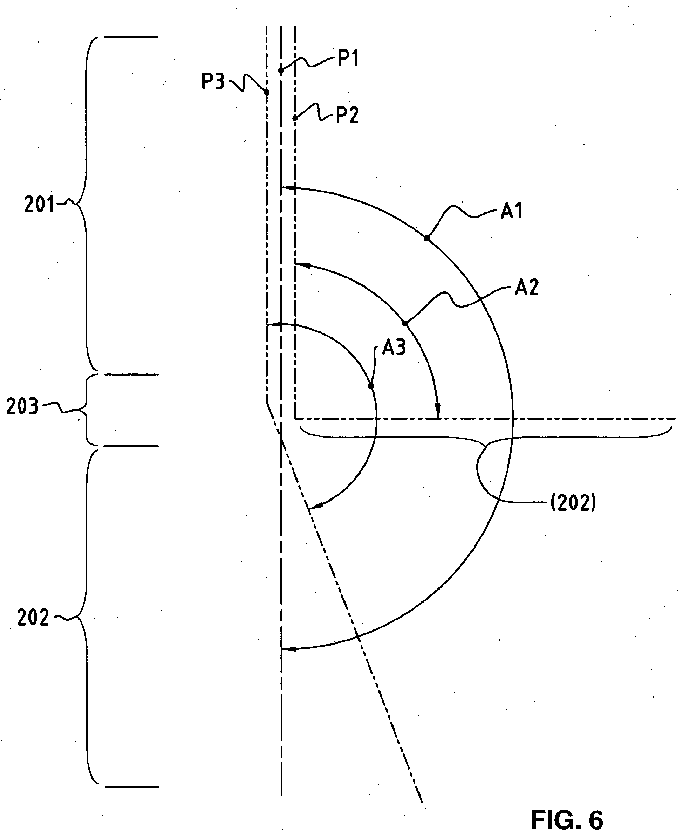

Figure 6 : une vue simplifiée montrant les différentes positions relatives de parties de la prothèse.

En se reportant au dessin, on voit un appareil 1 de liaison articulée pour une prothèse 2 de membre inférieur.

Classiquement, la prothèse 2 comprend :
- une première partie 201 capable d'assurer une fonction de cuisse,
- une seconde partie 202 capable d'assurer une fonction de jambe,
- une troisième partie 203 capable d'assurer une fonction de genou,
- une quatrième partie 204 capable d'assurer une fonction de pied en vue de l'appui sur un support 3.

L'appareil 1 de liaison articulé est incorporé à la troisième partie 203 de manière à assurer la liaison articulée entre la première partie 201 et la seconde partie 202, et ce, entre au moins deux positions relatives dont :
- une première position P1 dans laquelle la première partie 201 et la seconde partie 202 s'étendent sensiblement dans le prolongement l'une de l'autre, formant un premier angle A1, et
- une seconde position P2 dans laquelle la première partie 201 et la seconde partie 202 forment un second angle A2 prédéterminé correspondant à la flexion maximale admise par la prothèse 2.

Autrement dit, la première position P1 est la configuration d'extension de la prothèse 2, c'est-à-dire la configuration dans laquelle la première partie 201 et la seconde partie 202 forment un premier angle A1 qui est l'angle le plus ouvert qu'elles peuvent adopter.

En ce premier angle A1 la prothèse 2 permet à une personne (non représentée) de se tenir en station debout.

Egalement, la seconde position P2 est la configuration de flexion de la prothèse 2, c'est-à-dire la configuration dans laquelle la première partie 201 et la seconde partie 202 forment un second angle A2, qui est l'angle le plus fermé qu'elles peuvent adopter.

En ce second angle A2 la prothèse 2 permet à une personne (non représentée) de se tenir au moins en station assise, voire en station sensiblement accroupie.

Outre un mécanisme d'articulation (non représenté), l'appareil 1 de liaison articulé comprend un premier dispositif 1000 destiné à opposer une résistance prédéterminée au moins lors de la flexion de la prothèse 2, cette résistance étant commutée par ledit premier dispositif 1000 entre deux valeurs dont :
- une valeur maximale prédéterminée sélectionnée par défaut, et
- une valeur minimale prédéterminée sélectionnée après que, la première partie 201 et la seconde partie 202 étant situées dans une troisième position P3 voisine de la première position P1, ces parties sont sollicitées vers la première position P1 et déplacées en cette première position P1.

Lorsque la prothèse est portée par une personne (non représentée), c'est-à-dire que la première partie 201 est reliée à une partie subsistante de cuisse, la personne doit pouvoir modifier la configuration de la prothèse 2 et notamment :
- lever la première partie 201 pour contraindre la prothèse 2 à fléchir et tendre à placer ladite première partie 201 et la seconde partie 202 dans la seconde position P2,
- appuyer sur la première partie 201 pendant que la quatrième partie 204 est en contact avec un support 3 et tendre à placer ladite première partie 201 et la seconde partie 202 dans la première position P1.

La troisième position P3 est une position de la prothèse 2 qui est voisine de la configuration d'extension.

Dans la troisième position, la première partie 201 et la seconde partie 202 forment un troisième angle A3 qui est inférieur au premier angle A1 de quelques degrés, par exemple d'un virgule cinq degrés.

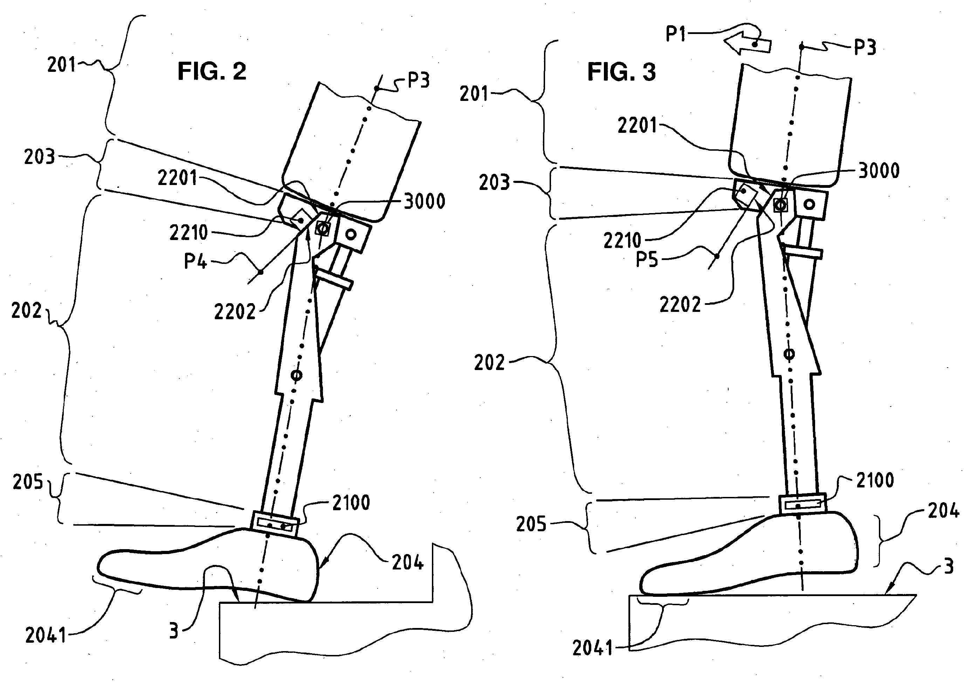

L'appareil 1 selon l'invention comprend un second dispositif 2000 comprenant au moins
- un premier moyen fonctionnel 2100 capable de détecter l'appui de la quatrième partie 204 sur un support 3, au moins par une première zone 2041 de cette quatrième partie 204 qui correspond à l'avant d'un pied, et
- un second moyen fonctionnel 2200 capable,
   d'entraver le déplacement de la première partie 201 et la seconde partie 202 vers la première position P1 lorsque,
      .. d'une part, elles sont situées dans une troisième position P3 voisine de la première position P1 et qu'elles sont sollicitées vers ladite première position P1 et,
      **..** d'autre part, le premier moyen fonctionnel 2100 ne détecte pas d'appui de la quatrième partie 204 sur un support 3 par ladite première zone 2041,
   . d'autoriser le déplacement de la première partie 201 et la seconde partie 202 vers la première position P1 lorsque, d'une part, elles sont situées dans une troisième position P3 voisine de la première position P1 et qu'elles sont sollicitées vers la première position P1 et, d'autre part, le premier moyen fonctionnel 2100 détecte l'appui de la quatrième partie 204 sur un support 3 par ladite première zone 2041 de la quatrième partie 204 (Cette dernière situation est illustrée en figure 3 et la possibilité de passage de la troisième position P3 à la première position P1 est symbolisée par une flèche repérée P1).

Par cela, la prothèse 2 ne peut pas fléchir librement et de manière préjudiciable à la personne qui la porte, particulièrement lorsque cette personne doit descendre une ou plusieurs marches en s'appuyant alternativement sur la prothèse 2.

En effet, le second moyen 2200 interdit que la prothèse 2 puisse librement fléchir lorsqu'elle est sollicitée vers la configuration d'extension, mais qu'aucun appui n'a été détecté au niveau de la première zone 2041 correspondant à l'avant d'un pied.

Cette situation correspond au cas où une personne cherche à trouver un appui sur une marche et au cas où la prothèse doit pouvoir fléchir en opposant une résistance suffisante pour compenser le poids de la personne.

Au contraire le second moyen 2200 autorise la libre flexion de la prothèse 2 après qu'elle ait atteint la configuration d'extension, ce qui permet à la seconde partie de s'articuler librement (ou avec une résistance réduite) lors d'une phase de la marche pendant laquelle la personne qui porte la prothèse ramène la prothèse vers l'avant pour effectuer un pas.

Le second moyen 2200 peut comprendre un processeur fonctionnant selon un programme préétabli et permettant le traitement de différentes informations fonctionnelles.

De manière avantageuse, la prothèse 2 de membre inférieur comprend une cinquième partie 205 destinée à assurer une fonction de cheville.

Par l'expression « fonction de cheville » on désigne au moins une fonction de liaison mécanique entre la seconde partie 202 et la quatrième partie 204 et, de préférence une liaison mécanique avec possibilité de flexion, voire possibilité d'articulation.

Le premier moyen fonctionnel 2100 est avantageusement, mais non limitativement situé au niveau de la cinquième partie 205 et est sensible aux sollicitations mécaniques que reçoit cette cinquième partie 205, de manière à détecter l'appui de la première zone 2041 de la quatrième partie 204 sur un support 3.

Le premier moyen fonctionnel 2100 est, par exemple, constitué par un capteur de type dit à « jauge de contrainte » et est fixé sur un élément structurel appartenant à la cinquième partie 205.

L'homme du métier est à même, sans faire preuve d'invention, de déterminer le type de composant le mieux adapté pour réaliser la fonction recherchée.

Le second dispositif 2000 comprend un troisième moyen fonctionnel 3000 capable :
- de détecter la position de la première partie 201 et de la seconde partie 202 dans un secteur angulaire de valeur prédéterminée au moins compris entre la première position P1 et la troisième position P3,
- d'informer le second moyen fonctionnel 2200 de la position relative de la première partie 201 et de la seconde partie 202 dans ce secteur angulaire ou à l'extérieur de ce secteur.

Par cela, le fonctionnement de l'appareil 1 est sécurisé.

Le troisième moyen fonctionnel 3000 est, par exemple, constitué par un capteur angulaire et est situé au niveau d'un axe d'articulation que comprend la troisième partie 203 en vue de la liaison articulée de la première partie 201 et de la seconde partie 202 de la prothèse.

L'homme du métier est à même, sans faire preuve d'invention, de déterminer le type de composant le mieux adapté pour réaliser la fonction recherchée.

Le second moyen fonctionnel 2200 comprend au moins :
- une première butée 2201 qui, au moins indirectement portée par la seconde partie 202 de la prothèse 2, adopte au moins la même troisième position P3 que ladite seconde partie 202,
- une seconde butée 2202 destinée à coopérer avec la première butée 2201,
- un premier organe fonctionnel 2210 pour guider la seconde butée 2202 au moins entre deux positions relatives dont,
   . une quatrième position P4 dans laquelle elle se trouve opposée à la première butée 2201 pour pouvoir coopérer avec cette dernière lorsqu'elle se présente dans la troisième position P3, de manière à entraver son déplacement vers la première position P1
   . une cinquième position P5 dans laquelle elle autorise le déplacement de la première butée 2201 vers la première position P1,
- un second organe fonctionnel 2220 pour immobiliser la seconde butée 2202 dans la quatrième position P4 lorsque le premier moyen fonctionnel 2100 ne détecte pas l'appui de la quatrième partie 204 sur un support 3 par ladite première zone 2041 de cette quatrième partie 204.

La première butée 2201 est par exemple portée par un élément structurel de la prothèse qui appartient à la deuxième partie 202.

La seconde butée 2202 est par exemple indirectement portée par un autre élément structurel qui appartient à la troisième partie 203.

L'homme du métier est à même, sans faire preuve d'invention, de déterminer la situation la plus appropriée pour la première butée 2201 et la seconde butée 2202.

De manière notable :
- le premier organe fonctionnel 2210,
   . consiste en un premier cylindre 2211 et premier piston 2212 dans lequel le premier piston 2212 porte la seconde butée 2202 et le premier cylindre 2211,
   . comprend une première chambre 2213 qui, fermée par le premier piston 2212, contient un fluide 2214 soumis à l'action du premier piston 2212 et est reliée à une capacité 2215 par une conduite 2216 de transfert de ce fluide 2214,
   . est constitué et disposé de manière telle que la seconde butée 2202 portée par le premier piston 2212 puisse être guidée au moins entre la quatrième position P4 et la cinquième position P5
   - le second organe fonctionnel 2220 est de type commandé et permettant le contrôle du passage du fluide 2214 dans la conduite 2216 de transfert de manière à pouvoir entraver le déplacement du premier piston 2212 ou autoriser ce déplacement.

Sur le dessin, on a symbolisé le fluide 2214 par des petits points.

Selon une forme de réalisation :
- le fluide 2214 est un fluide de type hydraulique et
- le second organe fonctionnel 2220 pour le contrôle du passage du fluide 2214 dans la conduite 2216 de transfert consiste en une valve commandée 2220A qui, interposée sur la conduite 2216 de transfert pour être traversée par le fluide 2214, comprend un obturateur 2217 mobile entre une première situation S1 s'opposant à la traversée du fluide 2214 et une seconde situation S2 autorisant la traversée dudit fluide 2214.

Selon une autre forme de réalisation :
- le fluide 2214 est un fluide de type magnéto rhéologique, et
- le second organe fonctionnel 2220 pour le contrôle du passage du fluide 2214 dans la conduite 2216 de transfert consiste en un solénoïde 2220B localement disposé autour de la conduite 2216 de manière à permettre,
   . par une action magnétique de figer localement le fluide 2214 en vue de provoquer l'obstruction de la conduite 2216, ou
   . en l'absence d'action magnétique d'autoriser le passage du fluide 2214 dans la conduite 2216.

Suivant une forme de réalisation, la première chambre 2213 abrite un premier élément élastique (non représenté) qui exerce sur le premier piston 2212 une action d'intensité au moins suffisante pour, en l'absence de contact entre la seconde butée 2202 et la première butée 2201, induire le déplacement du premier piston 2212 de la cinquième position P5 vers la quatrième position P4.

Selon une autre forme de réalisation, la capacité 2215 est de type opposant une résistance élastique à l'entrée de fluide 2214 et cette résistance élastique est de valeur au moins suffisante pour induire le déplacement du premier piston 2212 de la cinquième position P5 vers la quatrième position P4.

Cela signifie que la seconde butée 2202 portée par le premier piston 2212 peut avantageusement suivre la première butée 2201 dans ses déplacements pour ne lui interdire un déplacement que lorsque cela s'impose.

Suivant une forme de réalisation, la capacité 2215 est constituée par un second cylindre 2218 abritant un second piston 2219 ainsi qu'un second élément élastique 2221 qui sollicite le second piston 2219 de manière à ce qu'il s'oppose élastiquement à l'entrée de fluide 2214 dans la capacité 2215.

Bien que cela ne soit pas représenté, l'appareil 1 comprend une source d'énergie électrique, telle une batterie d'accumulateurs.

## Revendications

1. Appareil (1) de liaison articulée pour prothèse (2) de membre inférieur, cette prothèse (2) comprenant,
. une première partie (201) capable d'assurer une fonction de cuisse,
. une seconde partie (202) capable d'assurer une fonction de jambe,
. une troisième partie (203) capable d'assurer une fonction de genou,
. une quatrième partie (204) capable d'assurer une fonction de pied en vue de l'appui sur un support (3),
l'appareil (1) de liaison articulé étant incorporé à la troisième partie (203) de manière à assurer la liaison articulée entre la première partie (201) et la seconde partie (202), et ce, entre au moins deux positions relatives dont,
. une première position (P1) dans laquelle la première partie (201) et la seconde partie (202) s'étendent sensiblement dans le prolongement l'une de l'autre, formant un premier angle (A1), et
. une seconde position (P2) dans laquelle la première partie (201) et la seconde partie (202) forment un second angle (A2) prédéterminé correspondant à la flexion maximale admise par la prothèse (2),
ledit appareil (1) de liaison articulé comprenant un mécanisme d'articulation et un premier dispositif (1000) destiné à opposer une résistance prédéterminée au moins lors de la flexion de la prothèse (2), cette résistance étant commutée par ledit premier dispositif (1000) entre deux valeurs dont,
. une valeur maximale prédéterminée sélectionnée par défaut, et
. une valeur minimale prédéterminée sélectionnée après que, la première partie (201) et la seconde partie (202) étant situées dans une troisième position (P3) voisine de la première position (P1), ces parties sont sollicitées vers la première position (P1) et déplacées en cette première position (P1),
cet appareil (1) de liaison articulée étant **caractérisé en ce qu'**il comprend un second dispositif (2000) comprenant au moins :
- un premier moyen fonctionnel (2100) capable de détecter l'appui de la quatrième partie (204) sur un support (3), au moins par une première zone (2041) de cette quatrième partie (204) qui correspond à l'avant d'un pied, et
- un second moyen fonctionnel (2200) capable,
. d'entraver le déplacement de la première partie (201) et la seconde partie (202) vers la première position (P1) lorsque,
.. d'une part, elles sont situées dans une troisième position (P3) voisine de la première position (P1) et qu'elles sont sollicitées vers ladite première position (P1) et,
.. d'autre part, le premier moyen fonctionnel (2100) ne détecte pas d'appui de la quatrième partie (204) sur un support (3) par ladite première zone (2041),
. d'autoriser le déplacement de la première partie (201) et la seconde partie (202) vers la première position (P1) lorsque, d'une part, elles sont situées dans une troisième position (P3) voisine de la première position (P1) et qu'elles sont sollicitées vers la première position (P1) et, d'autre part, le premier moyen fonctionnel (2100) détecte l'appui de la quatrième partie (204) sur un support (3) par ladite première zone (2041) de la quatrième partie (204).

2. Appareil (1) selon la revendication 1 et de type destiné à équiper une prothèse (2) de membre inférieur comprenant une cinquième partie (205) destinée à assurer une fonction de cheville, **caractérisé en ce que** le premier moyen fonctionnel (2100) est situé au niveau de la cinquième partie (205) et est sensible aux sollicitations mécaniques que reçoit cette cinquième partie (205), de manière à détecter l'appui de la première zone (2041) de la quatrième partie (204) sur un support (3).

3. Appareil (1) selon l'une quelconque des revendications 1 à 2, **caractérisé en ce que** le second dispositif (2000) comprend un troisième moyen fonctionnel (3000) capable :
- de détecter la position de la première partie (201) et de la seconde partie (202) dans un secteur angulaire de valeur prédéterminée au moins compris entre la première position (P1) et la troisième position (P3),
- d'informer le second moyen fonctionnel (2200) de la position relative de la première partie (201) et de la seconde partie (202) dans ce secteur angulaire ou à l'extérieur de ce secteur.

4. Appareil (1) selon l'une quelconque des revendications 1 à 3, **caractérisé en ce que** le second moyen fonctionnel (2200) comprend au moins :
- une première butée (2201) qui, au moins indirectement portée par la seconde partie (202) de la prothèse (2), adopte au moins la même troisième position (P3) que ladite seconde partie (202),
- une seconde butée (2202) destinée à coopérer avec la première butée (2201),
- un premier organe fonctionnel (2210) pour guider la seconde butée (2202) au moins entre deux positions relatives dont,
. une quatrième position (P4) dans laquelle elle se trouve opposée à la première butée (2201) pour pouvoir coopérer avec cette dernière lorsqu'elle se présente dans la troisième position (P3), de manière à entraver son déplacement vers la première position (P1),
. une cinquième position (P5) dans laquelle elle autorise le déplacement de la première butée (2201) vers la première position (P1),
- un second organe fonctionnel (2220) pour immobiliser la seconde butée (2202) dans la quatrième position (P4) lorsque le premier moyen fonctionnel (2100) ne détecte pas l'appui de la quatrième partie (204) sur un support (3) par ladite première zone (2041) de cette quatrième partie (204).

5. Appareil selon la revendication 4, **caractérisé en ce que** :
- le premier organe fonctionnel (2210),
. consiste en un premier cylindre (2211) et premier piston (2212) dans lequel le premier piston (2212) porte la seconde butée (2202) et le premier cylindre (2211) comprend une première chambre (2213) qui, fermée par le premier piston (2212), contient un fluide (2214) soumis à l'action du premier piston (2212) et est reliée à une capacité (2215) par une conduite (2216) de transfert de ce fluide (2214),
. est constitué et disposé de manière telle que la seconde butée (2202) portée par le premier piston (2212) puisse être guidée au moins entre la quatrième position (P4) et la cinquième position (P5),
- le second organe fonctionnel (2220) est de type commandé et permettant le contrôle du passage du fluide (2214) dans la conduite (2216) de transfert de manière à pouvoir entraver le déplacement du premier piston (2212) ou autoriser ce déplacement.

6. Appareil selon la revendication 5, **caractérisé en ce que** :
- le fluide (2214) est un fluide de type hydraulique, et
- le second organe fonctionnel (2220) pour le contrôle du passage du fluide (2214) dans la conduite (2216) de transfert, consiste en une valve commandée (2220A) qui, interposée sur la conduite (2216) de transfert pour être traversée par le fluide (2214), comprend un obturateur (2217) mobile entre une première situation (S1) s'opposant à la traversée du fluide (2214) et une seconde situation (S2) autorisant la traversée dudit fluide (2214).

7. Appareil selon la revendication 5, **caractérisé en ce que** :
- le fluide (2214) est un fluide de type magnéto rhéologique, et
- le second organe fonctionnel (2220) pour le contrôle du passage du fluide (2214) dans la conduite (2216) de transfert consiste en un solénoïde (2220B) localement disposé autour de la conduite (2216) de manière à permettre,
. par une action magnétique de figer localement le fluide (2214) en vue de provoquer l'obstruction de la conduite (2216), ou
. en l'absence d'action magnétique d'autoriser le passage du fluide (2214) dans la conduite (2216).

8. Appareil selon l'une quelconque des revendications 5 à 7, **caractérisé en ce que** la première chambre (2213) abrite un premier élément élastique qui exerce sur le premier piston (2212) une action d'intensité au moins suffisante pour, en l'absence de contact entre la seconde butée (2202) et la première butée (2201), induire le déplacement du premier piston (2212) de la cinquième position (P5) vers la quatrième position (P4).

9. Appareil selon l'une quelconque des revendications 5 à 7, **caractérisé en ce que** la capacité (2215) est de type opposant une résistance élastique à l'entrée de fluide (2214) et cette résistance élastique est de valeur au moins suffisante pour induire le déplacement du premier piston (2212) de la cinquième position (P5) vers la quatrième position (P4).

10. Appareil selon la revendication 9, **caractérisé en ce que** la capacité (2215) est constituée par un second cylindre (2218) abritant un second piston (2219) ainsi qu'un second élément élastique (2221) qui sollicite le second piston (2219) de manière à ce qu'il s'oppose élastiquement à l'entrée de fluide (2214) dans la capacité (2215).

11. Prothèse (2) de membre inférieur équipée de l'appareil (1) selon l'une quelconque des revendications 1 à 10.

## Patentansprüche

1. Gelenkige Verbindungseinrichtung (1) für eine Schenkelprothese (2), wobei dies Prothese (2) aufweist
• einen ersten Bereich (201) geeignet zur Sicherstellung einer Oberschenkelfunktion,
• einen zweiten Bereich (202) geeignet zur Sicherstellung einer Beinfunktion,
• einen dritten Bereich (203) geeignet zur Sicherstellung einer Kniefunktion,
• einen vierten Bereich (204) geeignet zur Sicherstellung einer Fussfunktion für den Zweck des Abstellens auf einer Unterlage (3),
wobei die gelenkige Verbindungseinrichtung (1) in dem dritten Bereich (203) derart aufgenommen ist, dass die gelenkige Verbindung zwischen dem ersten Bereich (201) und dem zweiten Bereich (202) und dies zwischen wenigstens zwei relativen Positionen sichergestellt ist, welche sind:
• eine erste Position (P1), in welcher der erste Bereich (201) und der zweite Bereich (202) sich jeweils im Wesentlichen in der Verlängerung von einander erstrecken und einen ersten Winkel (A1) bilden und
• eine zweite Position (P2), in welcher der erste Bereich (201) und der zweite Bereich (202) einen zweiten vorbestimmten Winkel (A2) bilden, welche der maximal von der Prothese (2) zugelassenen Biegung entspricht,
wobei die gelenkige Verbindungseinrichtung (1) einen Gelenkmechanismus und eine erste Vorrichtung (1000), die zumindest während der Biegung der Prothese (2) einem vorbestimmten Wiederstand entgegen wirken soll, aufweist, wobei der Wiederstand von der ersten Vorrichtung (1000) zwischen zwei Werten umgeschaltet wird, welche sind:
• ein willkürlich ausgewählter vorbestimmter Maximalwert und
• ein vorbestimmter Minimalwert, der ausgewählt wird nachdem der erste Bereich (201) und der zweite Bereich (202), angeordnet in einer dritten Position (P3) anschliessend an die erste Position (P1), in Richtung der ersten Position (P1) gezogen und in die erste Position (P1) bewegt sind,
wobei die gelenkige Verbindungseinrichtung (1) dadurch charakterisiert ist, dass sie eine zweite Vorrichtung (2000) aufweist, die wenigstens umfasst:
- ein erstes Funktionsmittel (2100) geeignet zur Detektion des Abstellens des vierten Bereichs (204) auf einer Unterlage (3), wenigstens für eine erste Zone (2041) dieses vierten Bereichs (204), die einem Vorderbereich eines Fusses entspricht, und
- ein zweites Funktionsmittel (2200), das geeignet ist:
• die Bewegung des ersten Bereichs (201) und des zweiten Bereichs (202) in Richtung der ersten Position (P1) zu hemmen, wenn
• • einerseits diese in einer dritten Position (P3) anschliessend an die erste Position (P1) sind und diese in Richtung der ersten Position (P1) gezogen werden und
• • andererseits das erste Funktionsmittel (2100) das Abstellen des vierten Bereichs (204) auf einer Unterlage (3) für die erste Zone (2041) nicht detektiert,
• die Bewegung des ersten Bereichs (201) und des zweiten Bereich (202) in Richtung der ersten Position (P1) zu ermöglichen, wenn diese einerseits in einer dritten Position (P3) anschliessend an die erste Position (P1) sind und diese in Richtung der ersten Position (P1) gezogen sind und andererseits das erste Funktionsmittel (2100) das Abstellen des vierten Bereichs (204) auf einer Unterlage (3) für die erste Zone (2041) des vierten Bereichs (204) detektiert.

2. Einrichtung (1) nach Anspruch 1 und für den Zweck der Ausstattung einer Prothese (2) für einen Schenkel, die einen fünften Bereich (205) vorgesehen zur Sicherstellung einer Knöchelfunktion aufweist, **dadurch gekennzeichnet, dass** das erste Funktionsmittel (2100) auf einer Höhe des fünften Bereichs (205) angeordnet ist und auf mechanische Beanspruchungen, die auf den fünften Bereich (205) wirken derart reagiert, dass das Abstellen der ersten Zone (2041) des vierten Bereichs (204) auf einer Unterlage (3) detektiert ist.

3. Einrichtung (1) nach einem der Ansprüche 1 oder 2, **dadurch gekennzeichnet, dass** die zweite Vorrichtung (2000) ein drittes Funktionsmittel (3000) aufweist, das geeignet ist:
- die Position des ersten Bereichs (201) und des zweiten Bereichs (202) in einem Winkelbereich eines vorbestimmten Wertes, der wenigstens zwischen der ersten Position (P1) und der dritten Position (P3) gelegen ist, zu detektieren,
- das zweite Funktionsmittel (2200) über die relative Position des ersten Bereichs (201) und des zweiten Bereichs (202) in diesem Winkelbereich oder ausserhalb dieses Bereiches zu informieren.

4. Einrichtung (1) nach einem der Ansprüche 1 bis 3, **dadurch gekennzeichnet, dass** das zweite Funktionsmittel (2200) wenigstens aufweist:
- einen ersten Anschlag (2201), der wenigstens indirekt von dem zweiten Bereich (202) der Prothese (202) getragen ist und wenigstens die gleiche dritte Position (P3) wie der zweite Bereich (202) annimmt,
- einen zweiten Anschlag (2202), der für ein Zusammenwirken mit dem ersten Anschlag (2201) vorgesehen ist,
- ein erstes Funktionselement (2210) zur Führung des zweiten Anschlags (2202) zwischen wenigstens zwei relativen Positionen, welche sind:
• eine vierte Position (P4), in der er dem ersten Anschlag (2201) gegenüber liegt, um mit diesem Zusammenzuwirken, wenn er selbst in der dritten Position (P3) ist, derart dass seine Bewegung in Richtung der ersten Position (P1) gehemmt ist,
• eine fünfte Position (P5), in welcher er die Bewegung des ersten Anschlags (2201) in Richtung der ersten Position (P1) zulässt,
- ein zweites Funktionselement (2220), um den zweiten Anschlag (2202) in der vierten Position (P4) festzulegen, wenn das erste Funktionsmittel (2100) das Abstellen des vierten Bereichs (204) auf einer Unterlage (3) für die erste Zone (2041) dieses vierten Bereichs (204) nicht detektiert.

5. Einrichtung nach Anspruch 4, **dadurch gekennzeichnet, dass**:
- das erste Funktionselement (2210);
• aus einem ersten Zylinder (2211) und einem ersten Kolben (2212) besteht, wobei der erste Kolben (2212) den zweiten Anschlag (2202) trägt und der erste Zylinder (2211) eine erste Kammer (2213) aufweist, welche durch den ersten Kolben (2212) geschlossen ist und ein Fluid (2214) unter Einwirkung des ersten Kolbens (2212) beinhaltet und an einen Lagerraum (2215) über eine Transferleitung (2216) für dieses Fluid (2214) angeschlossen ist,
• derart ausgelegt und gestaltet ist, dass der von dem ersten Kolben (2212) getragene zweite Anschlag (2202) wenigstens zwischen der vierten Position (P4) und der fünften Position (P5) geführt werden kann,
- das zweite Funktionselement (2220) nach einer Art zur Steuerung und derart ausgebildet ist, dass es die Steuerung des Durchflusses des Fluides (2214) in der Transferleitung (2216) derart erlaubt, dass die Bewegung des ersten Kolbens (2212) gehemmt ist oder diese Bewegung ermöglicht ist.

6. Einrichtung nach Anspruch 5, **dadurch gekennzeichnet, dass**:
- das Fluid (2214) ein hydraulisches Fluid ist, und
- das zweite Funktionselement (2220) zur Steuerung des Durchflusses des Fluides (2214) in der Transferleitung (2216) aus einem gesteuerten Ventil (2220A) besteht, das in der Transferleitung (2216) angeordnet ist, um von dem Fluid (2214) durchströmt zu werden, und einen Absperrkörper (2217) aufweist, der zwischen einer ersten Stellung (S1), in der er den Durchfluss des Fluides (2214) verhindert, und einer zweiten Stellung (S2), in der er den Durchfluss des Fluides (2214) ermöglicht, beweglich ist.

7. Einrichtung nach Anspruch 5, **dadurch gekennzeichnet, dass**:
- das Fluid (2214) ein magnet-rheologisches Fluid ist und
- das zweite Funktionselement (2220) zur Kontrolle des Durchflusses des Fluides (2214) in der Transferleitung (2216) aus einem Solenoid (2220B) besteht, der örtlich derart um die Leitung (2216) angeordnet ist, dass es möglich ist:
- das Fluid (2214) durch ein magnetisches Einwirken lokal zu verdicken zum Zweck der Herstellung eines Verschlusses der Leitung (2216) oder
- in Abwesenheit eines magnetischen Einwirkens den Durchfluss des Fluides (2214) durch die Leitung (2216) zur ermöglichen.

8. Einrichtung nach einem der Ansprüche 5 bis 7, **dadurch gekennzeichnet, dass** in der ersten Kammer (2213) ein erstes elastisches Element aufgenommen ist, das auf den ersten Kolben (2212) mit einer Intensität einwirkt, die wenigstens ausreicht, um die Bewegung des ersten Kolbens (2212) aus der fünften Position (P5) in Richtung der vierten Position (P4) zu erwirken, wobei zwischen dem zweiten Anschlag (2202) und dem ersten Anschlag (2201) kein Kontakt besteht.

9. Einrichtung nach einem der Ansprüche 5 bis 7, **dadurch gekennzeichnet, dass** der Lagerraum (2215) derart ist, dass dem Einfliessen des Fluides (2214) ein elastischer Widerstand entgegengesetzt ist und dieser elastische Widerstand einen Wert aufweist der wenigstens ausreicht, die Bewegung des ersten Kolbens (2212) aus der fünften Position (P5) in Richtung der vierten Position (P4) aufzubringen.

10. Einrichtung nach Anspruch 9, **dadurch gekennzeichnet, dass** der Lagerraum (2215) aus einem zweiten Zylinder (2218) besteht, der sowohl einen zweiten Kolben (2219) als auch ein zweites elastisches Element (2221) aufnimmt, das den zweiten Kolben (2219) derart zieht, dass es selbst dem Einfliessen des Fluides (2214) in den Lagerraum (2215) elastisch entgegensteht.

11. Prothese (2) für einen Schenkel, die mit der Einrichtung (1) nach einem der Ansprüche 1 bis 10 ausgebildet ist.

## Claims

1. Hinged connecting apparatus (1) for a lower limb prosthesis (2), this prosthesis (2) comprising
• a first part (201) capable of ensuring a thigh function,
• a second part (202) capable of ensuring a leg function,
• a third part (203) capable of ensuring a knee function,
• a fourth part (204) capable of ensuring a foot function with a view to supporting it on a support (3),
the hinged connecting apparatus (1) being incorporated in the third part (203) in such a way as to ensure the hinged connection between the first part (201) and the second part (202), and this between at least two relative position which are:
• a first position (P1) in which the first part (201) and the second part (202) extend substantially in the prolongation one of the other, forming a first angle (A1), and
• a second position (P2) in which the first part (201) and the second part (202) form a second predetermined angle (A2) corresponding to the maximal flexion permitted by the prosthesis (2),
said hinged connecting apparatus (1) comprising an articulation mechanism and a first device (1000) intended to oppose a predetermined resistance at least during the flexion of the prosthesis (2), this resistance being switched by said first device (1000) between two values which are:
• a predetermined maximal value selected by default, and
• a predetermined minimal value selected after the first part (201) and the second part (202), being situated in a third position (P3) adjacent to the first position (P1), are pulled towards the first position (P1) and are moved into this first position (P1),
this hinged connecting apparatus (1) being **characterised in that** it comprises a second device (2000) including at least:
- a first functional means (2100) capable of detecting the bearing of the fourth part (204) on a support (3), at least by a first zone (2041) of this fourth part (204) which corresponds to the front of a foot, and
- a second functional means (2200) capable
• of impeding the movement of the first part (201) and the second part (202) towards the first position (P1) when,
•• on the one hand, they are situated in a third position (P3) adjacent to the first position (P1) and they are pulled towards said first position (P1) and
•• on the other hand, the first functional means (2100) does not detect the bearing of the fourth part (204) on a support (3) by said first zone (2041),
• of allowing the movement of the first part (201) and the second part (202) towards the first position (P1) when, on the one hand, they are situated in a third position (P3) adjacent to the first position (P1) and they are pulled toward the first position (P1) and, on the other hand, the first functional means (2100) detects the bearing of the fourth part (204) on a support (3) by said first zone (2041) of the fourth part (204).

2. Apparatus (1) according to claim 1 and of type intended to fit a prosthesis (2) for a lower limb comprising a fifth part (205) intended to ensure an ankle function, **characterised in that** the first functional means (2100) is situated at the level of the fifth part (205) and is sensitive to mechanical stresses which this fifth part (205) receives, in such a way as to detect the bearing of the first zone (2041) of the fourth part (204) on a support (3).

3. Apparatus (1) according to any one of the claims 1 to 2, **characterised in that** the second device (2000) comprises a third functional means (3000) capable:
- of detecting the position of the first part (201) and of the second part (202) in an angular sector of predetermined value at least contained between the first position (P1) and the third position (P3),
- of informing the second functional means (2200) about the relative position of the first part (201) and of the second part (202) in this angular sector or outside this sector.

4. Apparatus (1) according to any one of the claims 1 to 3, **characterised in that** the second functional means (2200) comprises at least:
- a first stop (2201) which, borne at least indirectly by the second part (202) of the prosthesis (2), adopts at least the same third position (P3) as said second part (202),
- a second stop (2202) intended to co-operate with the first stop (2201),
- a first functional element (2210) to guide the second stop (2202) at least between two relative positions which are:
• a fourth position (P4) in which it finds itself opposed to the first stop (2201) in order to be able to co-operate with the latter when it presents itself in the third position (P3), in such a way as to impede its movement towards the first position (P1),
• a fifth position (P5) in which it allows the movement of the first stop (2201) toward the first position (P1),
- a second functional element (2220) to immobilize the second stop (2202) in the fourth position (P4) when the first functional means (2100) does not detect the bearing of the fourth part (204) on a support (3) by said first zone (2041) of this fourth part (204).

5. Apparatus according to claim 4, **characterised in that**:
- the first functional element (2210),
• consists of a first cylinder (2211) and first piston (2212) in which the first piston (2212) bears the second stop (2202), and the first cylinder (2211) comprises a first chamber (2213), which, closed by the first piston (2212), contains a fluid (2214) under the action of the first piston (2212) and is connected to a volume (2215) by a transfer conduit (2216) for this fluid (2214),
• is made up and configured in such a way that the second stop (2202) borne by the first piston (2212) is able to be guided at least between the fourth position (P4) and the fifth position (P5),
- the second functional element (2220) is of controlled type and of type permitting the control of the passage of the fluid (2214) in the transfer conduit (2216) in such a way as to be able to impede the movement of the first piston (2212) or to allow this movement.

6. Apparatus according to claim 5, **characterised in that**:
- the fluid (2214) is a fluid of hydraulic type, and
- the second functional element (2220) for control of the passage of the fluid (2214) in the transfer conduit (2216) consists of a controlled valve (2220A) which, placed on the transfer conduit (2216) to be passed through by the fluid (2214), comprises an obstructor (2217) movable between a first situation (S1) opposing the passage of the fluid (2214) and a second situation (S2) allowing the passage of said fluid (2214).

7. Apparatus according to claim 5, **characterised in that**:
- the fluid (2214) is a fluid of magetorheological type, and
- the second functional element (2220) for control of the passage of the fluid (2214) in the transfer conduit (2216) consists of a solenoid (2220B) disposed locally about the conduit (2216) in such a way as to permit
• through a magnetic action, to coagulate the fluid (2214) locally with a view to causing the obstruction of the conduit (2216), or
• in the absence of magnetic action, to allow the passage of the fluid (2214) in the conduit (2216).

8. Apparatus according to any one of the claims 5 to 7, **characterised in that** the first chamber (2213) accommodates a first elastic element which exerts on the first piston (2212) an action of intensity at least sufficient to bring about, in the absence of contact between the second stop (2202) and the first stop (2201), the movement of the first piston (2212) from the fifth position (P5) toward the fourth position (P4).

9. Apparatus according to any one of the claims 5 to 7, **characterised in that** the volume (2215) is of type opposing an elastic resistance to the entry of fluid (2214), and this elastic resistance is of value at least sufficient to bring about the movement of the first piston (2212) from the fifth position (P5) towards the fourth position (P4).

10. Apparatus according to claim 9, **characterised in that** the volume (2215) is constituted by a second cylinder (2218) accommodating a second piston (2219) as well as a second elastic element (2221) which pulls the second piston (2219) in such a way that it opposes itself elastically to the entry of fluid (2214) in the volume (2215).

11. Prosthesis (2) for a lower limb provided with the apparatus (1) according to any one of the claims 1 to 10.
